# EUROPÄISCHE PATENTSCHRIFT

(11) **EP 1 080 112 B1**
(45) Veröffentlichungstag und Bekanntmachung des Hinweises auf die Patenterteilung: **30.07.2003**
(21) Anmeldenummer: 99927773.4
(22) Anmeldetag: 27.05.1999
(51) Int. Cl.: C07K 14/765, C07K 16/06, C07K 14/755, C12N 9/74, C12N 9/64, C07K 14/81

(54) **VERFAHREN ZUR TRENNUNG UND/ODER ISOLIERUNG VON PLASMAPROTEINEN MIT ANNULARER CHROMATOGRAPHIE**
METHOD FOR SEPARATING AND/OR ISOLATING PLASMA PROTEINS BY ANNULARCHROMATOGRAPHY
PROCEDE POUR SEPARER ET/OU ISOLER DES PROTEINES PLASMATIQUES PAR CHROMATOGRAPHIE ANNULAIRE

(30) Priorität: 27.05.1998 DE 19823814
(43) Veröffentlichungstag der Anmeldung: 07.03.2001
(73) Patentinhaber: Octapharma AG, 8853 Lachen (CH)
(72) Erfinder: Buchacher, Andrea, 1100 Wien (AT); Josic, Djuro, 1100 Wien (AT); Gruber, Gerhard, 1100 Wien (AT); PRIOR, Adalbert, A-6840 Götzis (AT); WOLFGANG, Jürgen, A-6840 Götzis (AT); IBERER, Günther, A-2322 Hennersdorf (AT)
(74) Vertreter: Meyers, Hans-Wilhelm, Dr.Dipl.-Chem.
(86) Internationale Anmeldenummer: EP9903659
(87) Internationale Veröffentlichungsnummer: WO99061475

(56) Entgegenhaltungen:
- WO-A-99/28740
- K REISSNER ET AL.: "Preparative desalting of bovine serum albumin by continuous annular chromatography " JOURNAL OF CHROMATOGRAPHY A., Bd. 763, Nr. 1-2, 1997, Seiten 49-56, XP002116640 ELSEVIER SCIENCE., NL ISSN: 0021-9673 in der Anmeldung erwähnt
- CHEMICAL ABSTRACTS, vol. 121, no. 13, 26. September 1994 (1994-09-26) Columbus, Ohio, US; abstract no. 152412, Y TAKAHASHI ET AL.: "Continuous separation and concentration of proteinbs using an annular chromatograph" XP002116642 & STUD. SURF. SCI CATAL., Bd. 80, 1993, Seiten 639-646,
- CHEMICAL ABSTRACTS, vol. 121, no. 14, 3. Oktober 1994 (1994-10-03) Columbus, Ohio, US; abstract no. 166938, G F BLOOMINGBURG & G CARTA: "Separation of protein mixtures by continuous annular chromatography with step elution" XP002116643 & CHEM. ENG.J., Bd. 55, Nr. 1-2, 1994, Seiten 19B-27B, lausanne
- G F BLOOMINGBURG ET AL.: "Continuous separation of proteins by annular chromatography" INDUSTRIAL & ENGINEERING CHEMISTRY RESEARCH., Bd. 30, Nr. 5, Mai 1991 (1991-05), Seiten 1061-1067, XP002116641 AMERICAN CHEMICAL SOCIETY. WASHINGTON. 261987 1, US ISSN: 0888-5885 in der Anmeldung erwähnt
- Y TAKAHASHI & S GOTO: "Continuous separation and concentration of proteins using the annular chromatograph" JOURNAL OF CHEMICAL ENGINEERING OF JAPAN., Bd. 25, Nr. 4, April 1992 (1992-04), Seiten 403-407, XP000332075 SOCIETY OF CHEMICAL ENGINEERS. TOKYO., JP ISSN: 0021-9592

## Beschreibung

Gegenstand der vorliegenden Erfindung ist ein Verfahren zur Trennung und/oder Isolierung von Plasmaproteinen aus einer Blutplasma oder virusinaktivierte Plasmaproteine enthaltenden Mischung.

Plasmaproteine spielen eine wichtige Rolle bei vielen physiologischen Vorgängen. So sind zum Beispiel die vitamin-K-abhängigen Plasmaglycoproteine maßgeblich an der Blutgerinnungskaskade beteiligt.

Die Gewinnung der Plasmaproteine stellt mithin ein bedeutendes technisches Verfahren dar. Das Ausgangsprodukt zur Herstellung der Plasmaproteine ist bereits relativ wertvoll, da es beispielsweise aus Blutspenden gewonnen wird. Es ist wirtschaftlich wie ethisch bedeutsam, Verfahren bereitzustellen, die die gewünschten Plasmaproteine in hoher Ausbeute und Aktivität bereitstellen. Plasmaproteine werden heute üblicherweise neben rekombinanten Herstellungsweisen aus Blutplasma gewonnen, indem übliche chromatographische Verfahren angewendet werden.

Es wurde gefunden, daß hochmolekulare Substanzen innerhalb einer Größenordnung mit der annularen Chromatographie zu trennen und zu isolieren sind. Es wurde weiter gefunden, daß humane plasmatische Proteine auch aus einer komplexen Mischung in überraschend einfacher Weise in gereinigter Form gewonnen werden können. Dies erfolgt mit einem Verfahren, das die folgenden Schritte umfaßt: die insbesondere humanen Plasmaproteine enthaltenden Mischung wird auf ein annular angeordnetes Trennmedium aufgegeben , auf dem eine Schicht Auftragsmittel aus sphärischen Partikeln, die eine hydrophobe Oberfläche aufweisen, aufgebracht ist. Das annular angeordnete Trennmedium wird im wesentlichen senkrecht um eine in Fließrichtung der Mischung durch das annular angeordnete Trennmedium definierten Achse in Rotation versetzt. Ein Elutionsmittel wird durch das annular angeordnete Trennmedium geleitet und am Ende des annular angeordneten Trennmediums austretende Fraktionen werden gesammelt.

Die Anwendung der annularen Chromatographie zur Entsalzung von Rinderserumalbumin enthaltenden Mischungen wurde bereits von K. Reissner et al. in Journal of Chromatography A, 763 (1997) 49 bis 56 beschrieben. Dabei stellten Reissner et al. darauf ab, daß für den Erfolg der chromatographischen Reinigung die gewaltigen Unterschiede in der Größe der zu trennenden Substanzen, nämlich Rinderserumalbumin und niedermolekulare Salze, entscheidend sei. Von Bloomingburg et al (Ind. Eng. Chem. Res. 30, 1061-1067 (1991)) wird gezeigt, daß eine Mischung von den reinen Komponenten bovines Serumalbumin und bovines Hämoglobin mittels eines annular angeordneten Kationenaustauschers getrennt werden können. Dabei wird mit einem einzigen Elutionsmittel unter isokratischen Bedingungen eluiert. Zum Aufbringen von Probenmaterial wird mit mehreren Auftragsvorrichtungen gearbeitet.

Bevorzugt wird die in der genannten Veröffentlichung beschriebene Vorrichtung zur Durchführung des erfindungsgemäßen Verfahrens eingesetzt. Die Figur 1 zeigt schematisch eine entsprechende Vorrichtung. Die Figur 2 zeigt ein typisches Chromatogramm eines Komplexes von Faktor VIII und von Willebrand Faktor und die Figur 3 zeigt eine entsprechende Chromatographie von Faktor IX. Figur 4 zeigt eine Trennung von BSA und IgG. Figur 5 zeigt das Ergebnis einer konventionellen säulenchromatographischen Trennung.

Als Quelle der zu trennenden und/oder isolierenden Plasmaglycoproteine wird Blutplasma oder virusinaktivierte Plasmaproteine enthaltende Mischungen eingesetzt. Dabei wird die plasmaproteinhaltige Mischung z.B. in üblicher Weise aufgearbeitet. Zur Virusinaktivierung werden insbesondere Verfahren eingesetzt, die als Solvent/Detergent-Verfahren bekannt sind. So wird eine solche Methode insbesondere in B. Horowitz et al. "Blood" 79 (1992) 826 bis 831 beschrieben.

Daher kann die Mischung auch ein Detergenz enthalten, welches zudem auch unerwünschte unspezifische Wechselwirkungen der Plasmaproteine mit den Trennmedien unterbinden kann. Die eingesetzte Mischung kann nicht nur aus Blutplasma erhalten werden, sondern auch als Fraktion einer Zellkultur zur Verfügung gestellt werden, welche die humanen Plasmaproteine enthält, die gentechnisch hergestellt worden sind.

Das annular angeordnete Trennmedium besteht vorzugsweise aus Materialien für die Adsorptionschromatographie, wie die der Ionenaustauscher-, Gelpermeations- bzw. Molekularausschluß-, Affinitäts-Chromatographie oder Chromatographie, die auf hydrophoben Wechselwirkungen beruht. Dafür werden die üblichen Materialien eingesetzt.

Es kann auch bevorzugt sein, daß mehr als ein Trennmedium in einer Chromatographieanordnung eingesetzt werden. So kann eine Chromatographiesäule mit Schichten aus verschiedenen Trennmedien befüllt werden, um einen kombinierten Trennungseffekt zu erzielen. Dabei können nicht nur gleichartige Medien gewählt werden, sondern sehr unterschiedliche Medien, z.B ein Anionenaustauscher und ein Medium für die hydrophobe Interaktionschromatographie, oder zwei Anionenaustauscher unterschiedlicher Stärke, oder ein Adsorptionsmaterial und ein Material für die Gelpermeationschromatographie. Es sollte aber darauf geachtet werden, daß keine unerwünschte Vermischung der Materialien erfolgt und daß die verwendeten Puffer für alle eingesetzten Trennmaterialien geeignet sind.

Ein bevorzugtes Trennmedium wird als ein kompaktes Blockmaterial (Monolith) eingesetzt, welches bereits z.B. eine für die Trennsäule geeignete Ringform aufweist. Dieses kann beispielsweise mit einer losen Schüttung eines Chromatographiematerials kombiniert werden. Die als Trennmedium geeigneten blockartig geformten Medien sind nicht nur anorganische oder organische Monolithen, die durch Blockpolymerisierung erhältlich sind, und z.B. in der EP-A-0 320 023, auf die ausdrücklich Bezug genommen wird, offenbart sind. Weiter sind auch Trägermaterialien wie z.B. geformte Membranen oder solche mit Textilstruktur, etwa auf Basis von Zellulose, für die annulare Chromatographie verwendbar. Dabei sind gegebenenfalls die Monolithen bzw. die Trägermaterialien an ihrer Oberfläche zum Erhalt von eventuell benötigten Liganden modifiziert.

Die Erfindung betrifft die günstige Aufbringung des Probenmaterials auf das Trennmedium. Es hat sich herausgestellt, dass die Verwendung eines Auftragemittels zur gezielten, lokalen Aufbringung des Probenmaterials bevorzugt ist. Dabei kann jedes erfindungsgemäße Material eingesetzt werden, welches die Probe kurzzeitig lokalisiert, und welches nicht mit dem Trennmedium vermischt ist. Dies kann durch eine spezielle Auswahl des Materials gewährleistet werden, wobei die Dichte und die Beschaffenheit der Materialoberfläche für die Auswahl eine Rolle spielen. Es ist aber auch möglich, eine Vorrichtung einzusetzen, die eine Trennschicht zwischen dem Auftragemittel und dem Trennmedium vorsieht. Als Auftragemittel werden sphärische Partikeln eingesetzt, z.B. Glasperlen, wobei die Partikel gegebenenfalls so behandelt sind, daß unspezifische Wechselwirkungen verhindert werden.

Die sphärischen Partikel sind mit einer hydrophoben Oberfläche zu versehen, sofern diese von vorneherein keine hinreichend hydrophobe Oberfläche aufweisen. Beispielsweise können Glaspartikel verwendet werden, die mit Reagenzien, wie Silanierungsreagenzien, hydrophobisiert wurden. Die Partikelgröße der Glaspartikel liegt vorzugsweise im Bereich von 20 bis 500 µm. Die sphärischen Partikel bedecken das annular angeordnete Trennmedium und schützen es vor mechanischen Einflüssen, die durch die Aufgabevorrichtung während der Rotation der das annular angeordnete Trennmedium haltenden Vorrichtung hervorgerufen werden. Sofern bereits relativ hydrophobe Oberflächen, wie sie beispielsweise durch Kunststoffpartikel entsprechender Art bereitgestellt werden, eingesetzt werden, ist es nicht notwendig deren Oberflächen zu hydrophobisieren.

Als Kunststoffe, aus denen die sphärischen Partikeln bestehen, kommen insbesondere Polymethacrylate und Polystyrol-Divinylbenzol in Frage.

Die mit dem erfindungsgemäßen Verfahren gewinnbaren Plasmaproteine sind insbesondere Inter-α-trypsininhibitor, Immunglobuline, wie IgG, Humanserumalbumin oder Glykoproteine aus der Gerinnungskaskade. Bevorzugt werden Vitamin-Kabhängige Faktoren der Blutgerinnungskaskade, wie der Faktor IX, weitere Blutgerinnungfaktoren, wie die Faktoren VIII, XI und XIII, Antithrombin III, α₁-Antitrypsin und Thrombin aus einer Plasmafraktion gewonnen. Der Faktor VIII wird beispielsweise durch Anionenaustauschchromatographie aus einer Fraktion des Kryopräzipitates von den begleitenden Proteinen, wie Fibrinogen, gegebenenfalls kombiniert mit der Molekularausschlußchromatographie, abgetrennt. Der Faktor IX wird in einer Mischung mit Vitamin K abhängigen Plasmaproteinen als Ausgangsmaterial eingesetzt, wobei die begleitenden Plasmaproteine effektiv mittels einer Kombination von Anionenaustausch- und Affinitätschromatographie bzw. Molekularausschlußchromatographie, aber auch hydrophober Interaktionschromatographie abgetrennt werden können.

gegenstand der vorliegenden Erfindung ist der Einsatz von humanen Plasmaproteinen, welche an sich mit großer Sorgfalt aus einem komplexen Proteingemisch gereinigt werden müssen. Dabei ist zu beachten, daß insbesondere die Humanproteine in möglichst geringem Umfang aktiviert bzw. denaturiert werden dürfen und die Mischung der humanen Plasmaproteine schwierig zu trennen ist, aufgrund der ähnlichen physikalisch-chemischen Eigenschaften der humanen Plasmaproteine. Es hat sich als vorteilhaft herausgestellt, daß das erfindungsgemäße Verfahren vor allem zum Trennen von mindestens zwei verschiedenen Humanproteinen eingesetzt werden kann, ohne deren biologische Aktivität wesentlich zu beeinflussen.

Bedingt durch die komplexen Ausgangsmaterialien muß auch oft eine große Proteinmenge aufgetragen und getrennt werden. Dabei ist die kontinuierliche Verfahrensweise von großem Vorteil, da die Kapazität der Säule praktisch unbegrenzt genutzt werden kann. Mit der annularen Anordnung kann erstmals eine real kontinuierliche Verfahrensweise zur Verfügung gestellt werden, die nicht nur das Aufbringen der Probe, die Trennung der Plasmaproteine und das Fraktionieren zur gleichen Zeit ermöglicht. Es kann sogar simultan das Trennmedium regeneriert und äquilibriert werden. Während ein Bereich des Trennmediums regeneriert wird, kann ein nächster Bereich mit einem Puffer äquilibriert werden, welcher dann für die Probenaufbringung in kontinuierlicher Weise eingesetzt wird. So kann die Chromatographieanlage im Dauereinsatz über Tage und Wochen genutzt werden. Dadurch kann die Kapazität der Säule effektiv erhöht werden, abhängig von der Dauer des kontinuierlichen chromatographischen Prozesses. Dies ist vor allem für die Gelpermeations-bzw. Molekularausschlußchromatographie von Vorteil, da diese Art der Chromatographie gemäß Stand der Technik durch die relativ geringe Kapazität zur Trennung von Proteinen begrenzt ist. Diese echte kontinuierliche Verfahrensweise unterscheidet sich auch grundlegend von anderen quasi kontinuierlichen säulenchromatographischen Methoden, in denen eine Reihe von physisch separierten Kompartimenten mit Trennmedium zum Einsatz kommen ("Simulating Moving Bed").

Die Rotationsgeschwindigkeit ist vorzugsweise im Bereich von 100 bis 2000 Grad/Stunde, vorzugsweise 600 bis 2000 Grad/Stunde gewählt. Dabei konnte bemerkt werden, daß bei den höheren Geschwindigkeiten die Fraktionierung vorteilhaft ist und die Verweildauer der Humanproteine im Trennmedium minimiert werden kann. Üblicherweise werden für die Trennung Durchflußraten im Bereich von 1 bis 2000, vorzugsweise 15 bis 300, cm/min gewählt.

Das erfindungsgemäße Verfahren eignet sich vor allem für den großtechnischen Einsatz zum präparativen Trennen von humanen Plasmaproteinen. So können in Abhängigkeit der spezifischen Aktivität der Proteine Ausbeuten von mindestens 40 bis nahezu 100% erhalten werden. Wenn von vorgereinigten Proteinen ausgegangen wird, kann sogar die weitere Reinigung als "polishing" mit einer Ausbeute von mindestens 90%, vorzugsweise mindestens 95% erzielt werden. Damit können in wirtschaftlicher Weise pharmazeutische Präparate zur Verfügung gestellt werden, welche insbesondere humane Plasmaproteine in hochgereinigter Form enthalten.

Desweiteren besitzt die annulare Chromatographie den Vorteil, daß sie kontinuierlich durchgeführt werden kann.

Das Trennen von humanen Plasmaproteinen erfolgt besonders gut bei der Adsorptionschromatographie unter Einsatz eines Stufengradienten und Verwendung von mindestens zwei verschiedenen Elutionslösungen unterschiedlicher Elutionsstärke. Es hat sich auch als vorteilhaft herausgestellt nur eine einzige Vorrichtung zum Auftragen des Probenmaterials einzurichten, um das Trennmedium mit dem Probenmaterial der hohen Proteinkonzentration nicht zu überlasten.

Zur Kontrolle der Fraktionierung und zur gezielten Auswahl der zu sammelnden Fraktionen wird vorteilhafterweise ein Monitor eingesetzt, der die Proteinkonzentration in den Eluaten kontinuierlich überwacht. Dazu kann ein geeigneter Detektor, beispielsweise ein Photometer eingesetzt werden.

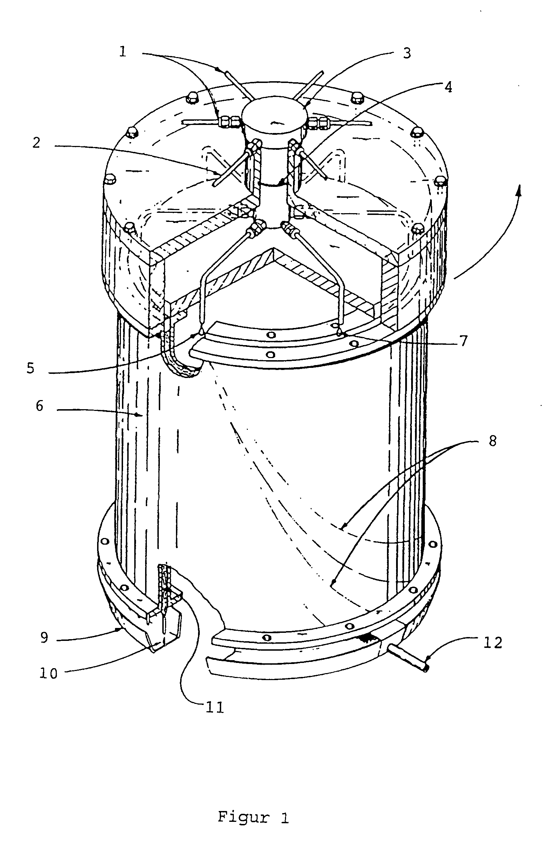

Die Figur 1 zeigt schematisch eine bevorzugte Vorrichtung zur Durchführung des erfindungsgemäßen Verfahrens. Das annular angeordnete Trägermaterial ist zwischen zwei konzentrischen Zylindern angeordnet. Der äußere Zylinder ist am oberen Ende mit einem Flansch verschlossen. Vorzugsweise ist der Zylinder selbst aus einem Stahl-Material gefertigt. Der innere Zylinder ist vorzugsweise aus beständigem Material gefertigt und kürzer als der äußere, so daß sich am oberen Ende ein Zwischenraum ergibt, der es erlaubt, das Elutionmittels gleichmäßig auf dem gesamten annular angeordneten Trennmedium zu verteilen. Am Kopf der chromatographischen Einheit wird die Probenaufgabevorrichtung und die Elutionmittelauftragevorrichtung angeordnet. Am Fuß der Einheit sind die beiden Zylinder mit einem zweiten Flansch verbunden. Dieser Flansch enthält eine Anzahl von Löchern, die im gleichmäßigen Abstand voneinander angeordnet sind, vorzugsweise sind die Löcher gleichmäßig in einem Winkel von mindestens 2° bis 180°-Intervallen, vorzugsweise 4 bis 36°-Intervallen verteilt. In die Löcher sind vorzugsweise flexible Kapillaren eingelassen, welche z.B. zu einem Fraktionssammler geführt werden.

Im einzelnen zeigt Figur 1 einen Elutionsmitteleinlaß 1, einen Probeneinlaß 2, einen stationären Einlaßverteiler 3, einen Einlaßdruckverschluß 4, einen kontinuierlichen Probenstrom 5, eine rotierende annulare Chromatographie-Einheit 6, einen Elutionsmittelfluß 7, eine aufgetrennte Probe 8, eine stationäre Sammeleinrichtung für den Elutionsmittelabfall 9, einen Elutionsmittelausgang 10, ein annular angeomdetes Trägermaterial 11 und einen Produktauslaß 12.

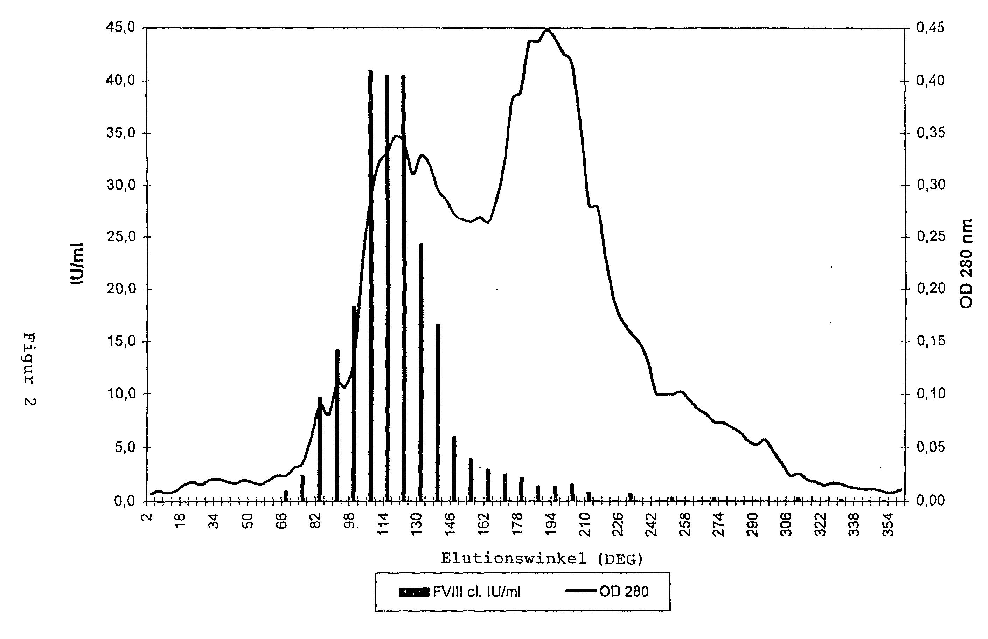

Figur 2 zeigt eine annulare Chromatographie von Faktor VIII/von Willebrandt Faktor.

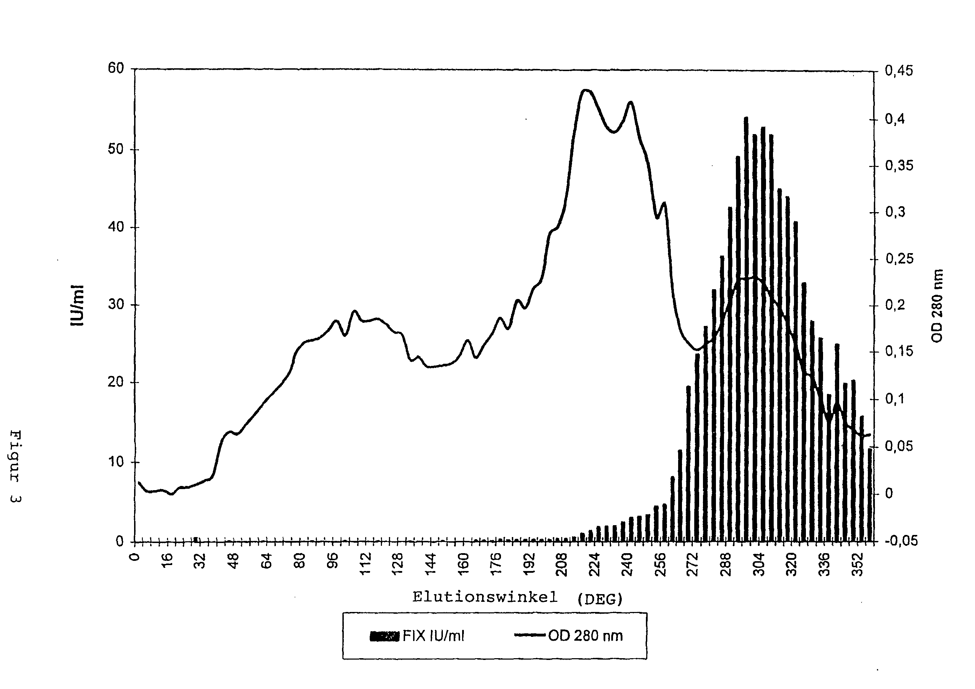

Figur 3 zeigt eine annulare Chromatographie von Faktor IX.

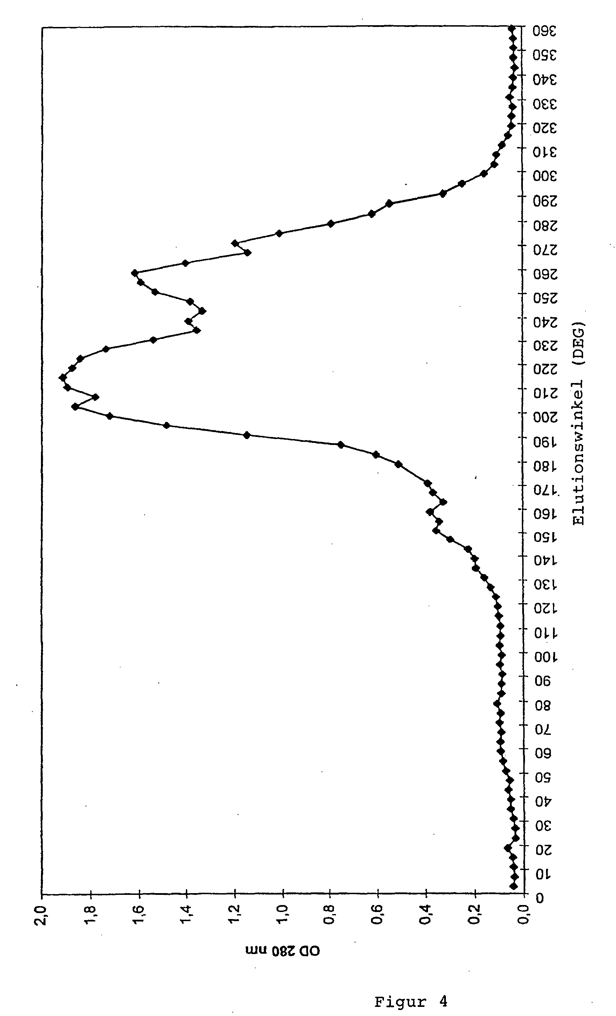

Figur 4 zeigt eine annulare Chromatographie von Immunglobulin G und Rinderserumalbumin (BSA), wobei Immunglobulin G zuerst eluiert.

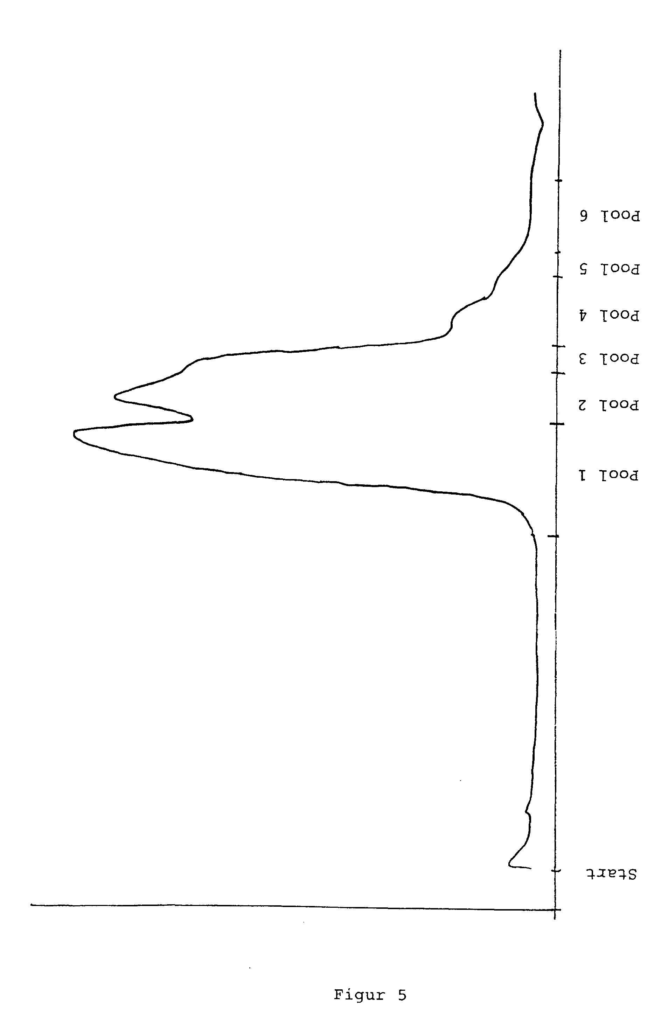

Figur 5 zeigt zum Vergleich einen Ausschlußchromatographie (size exclusion chromatography) eines Faktor VIII Konzentrats auf einer konventionellen diskontinuierlichen axialen Säule.

### Beispiel 1 (Vergleichsbeispiel)

Eine annulare Chromatographie-Vorrichtung gemäß Journal of Chromatography A, 563 (1997), 49 bis 56 wurde mit 2 1 Fractogel BioSec EMD 650 (S) gepackt. Die Dauer der Aufbringung der Mischung, die getrennt werden soll, hängt von der Elutionsflußrate ab. Nach Passieren des Bodens des annular angeordneten Trägermediums wurde die Probenaufbringung für eine weitere Stunde durchgeführt um ein Gleichgewicht zu erreichen. Danach wurde die Fraktionsammlung gestartet. Die in den Beispielen verwendete Apparatur besaß im Abstand von jeweils 2° einen Auslaß am Boden der Chromatographie-Einheit. Zwei Auslaßöffhungen wurden jeweils zu einer Fraktion zusammengefaßt.

### Trennung von humanem polyklonalen IgG und BSA

Zu trennende Mischung: 2,5 mg/ml IgG und 5 mg/ml BSA in aqua injectabilia.

Puffer:
27,5 mM Dinatriumhydrogenphosphat-dihydrat
12,5 mM Natriumdihydrogenphosphat-dihydrat
0,2 mM Natriumchlorid
pH = 7,2

Das Trennungsergebnis ist in Figur 4 gezeigt.

### Beispiel 2 (Vergleichsbeispiel)

### Trennung von Faktor IX Konzentrat

Probenlösung: Faktor IX Lyophilisate mit 500 IU (Octanyne, Octapharma GmbH) wurden gelöst und auf eine Konzentration von 20 bis 500 IU/ml eingestellt.

Puffer:
20 mM Natriumcitrat
0,2 M Natriumchlorid
2 mM Calciumchlorid,
pH = 7,4

Das Chromatogramm der Trennung ist in Figur 3 gezeigt.

### Beispiel 3 (Vergleichsbeispiel)

### Trennung von Faktor VIII Konzentrat

Zu trennende Mischung Faktor VIII Lyophilisat mit 500 IU (Emoclot) wurden gelöst und auf eine Konzentration von 20 bis 500 IU/ml eingestellt.

Puffer:
20 mM Natriumcitrat
0,2 M Natriumchlorid
2 mM Calciumchlorid
pH = 7,4

Das Chromatogramm der Trennung ist in Figur 2 gezeigt.

## Patentansprüche

1. Verfahren zur Trennung und/oder Isolierung von humanen Plasmaproteinen aus einer Blutplasma oder virusinaktivierte Plasmaproteine enthaltenden Mischung, wobei
- die Mischung auf ein annular angeordnetes Trennmedium aufgegeben wird, auf dem eine Schicht Auftragsmittel aus sphärischen Partikeln, die eine hydrophobe Oberfläche aufweisen, aufgebracht ist,
- das annular angeordnete Trennmedium im wesentlichen senkrecht um eine in Fließrichtung der Mischung durch das annular angeordnete Trennmedium definierten Achse rotiert,
- ein Elutionsmittel durch das annular angeordnete Trennmedium geleitet wird und
- am Ende des annular angeordneten Trennmediums austretende Fraktionen gesammelt werden.

2. Verfahren nach Anspruch 1, **dadurch gekennzeichnet, dass** das Auftragsmittel sphärische Partikel umfasst.

3. Verfahren nach Anspruch 1 und/oder 2, **dadurch gekennzeichnet, daß** die Mischung mindestens zwei zu trennende Plasmaproteine enthält.

4. Verfahren nach Anspruch 1 bis 3, wobei das annular angeordnete Trennmedium Ionenaustauscher-, Gelpermeations- bzw. Molekularausschlussoder Affinitäts-Chromatographie und Chromatographie, die auf hydrophoben Wechselwirkungen beruht, ist.

5. Verfahren- nach mindestens einem der Ansprüche 1 bis 4, wobei die Plasmaproteine Inter-α-trypsininhibitor, α₁-Antitrypsin, Antithrombin III, Immunglobuline, wie IgG, Humanserumalbumin oder Glykoproteine vorzugsweise aus der Gerinnungskaskade oder Vitamin-K-abhängigen Faktoren der Blutgerinnung sind.

6. Verfahren nach mindestens einem der Ansprüche 1 bis 4, **dadurch gekennzeichnet, dass** die Plasmaproteine ausgesucht sind aus den Blutgerinnungsfaktoren VIII, IX und Thrombin.

7. Verfahren nach einem der Ansprüche 1 bis 6, **dadurch gekennzeichnet, dass** die Aufgabe der Mischung, die Trennung der Plasmaproteine und die Fraktionierung kontinuierlich vorgenommen wird.

8. Verfahren nach einem der Ansprüche 1 bis 7, **dadurch gekennzeichnet, dass** gleichzeitig mit der Trennung der Plasmaproteine das Trennmedium kontinuierlich regeneriert und äquilibriert wird.

9. Verfahren nach einem der Ansprüche 1 bis 8, **dadurch gekennzeichnet, dass** bei Verwendung eines Materials zur Adsorptionschromatographie als Trennmedium mindestens zwei verschiedene Elutionsmittel gleichzeitig durch das annular angeordnete Trennmedium geleitet werden.

10. Verfahren nach einem der Ansprüche 1 bis 9, **dadurch gekennzeichnet, dass** mindestens zwei verschiedene Trennmedien in Schichten eingesetzt werden.

11. Verfahren nach einem der Ansprüche 1 bis 10, **dadurch gekennzeichnet, dass** als Trennmedium ein polymeres Blockmaterial eingesetzt wird.

## Claims

1. A process for the separation and/or isolation of human plasma proteins from a mixture containing blood plasma or virus-inactivated plasma proteins, wherein
- said mixture is applied to a separation medium having an annular design to which a layer of an application medium consisting of spherical particles having a hydrophobic surface has been applied;
- said separation medium having the annular design is rotated essentially vertically about an axis which is defined in the direction of flow of the mixture through the separation medium having the annular design;
- an eluent is passed through the separation medium having the annular design; and
- fractions exiting at the end of the separation medium having the annular design are collected.

2. The process according to claim 1, **characterized in that** said application medium comprises spherical particles.

3. The process according to claims 1 and/or 2, **characterized in that** said mixture contains at least two plasma proteins to be separated.

4. The process according to claims 1 to 3, wherein said separation medium having the annular design is used for ion-exchange, gel permeation, molecular size exclusion or affinity chromatography or chromatography based on hydrophobic interactions.

5. The process according to at least one of claims 1 to 4, wherein said plasma proteins are inter-α-trypsin inhibitor, α₁-antitrypsin, antithrombin III, immune globulins, such as IgG, human serum albumin or glycoproteins, preferably from the clotting cascade, or vitamin K dependent blood clotting factors.

6. The process according to at least one of claims 1 to 4, **characterized in that** said plasma proteins are selected from blood clotting factors VIII, IX, and thrombin.

7. The process according to any of claims 1 to 6, **characterized in that** the functions of mixing, of separating the plasma proteins and of the fractioning are performed continuously.

8. The process according to any of claims 1 to 7, **characterized in that** the separation medium is continuously regenerated and equilibrated, simultaneously with the separation of the plasma proteins.

9. The process according to any of claims 1 to 8, **characterized in that** when a material for adsorption chromatography is used as the separation medium, at least two different eluents are simultaneously passed through said separation medium having the annular design.

10. The process according to any of claims 1 to 9, **characterized in that** at least two different separation media are employed in layers.

11. The process according to any of claims 1 to 10, **characterized in that** a polymeric block material is employed as said separation medium.

## Revendications

1. Procédé de séparation et/ou d'isolation de protéines de plasma humaines à partir d'un mélange contenant du plasma sanguin ou des protéines de plasma inactivées vis vis des virus, au cours duquel
- le mélange est introduit sur un milieu de séparation disposé de façon annulaire, sur lequel est appliquée une couche d'agent de revêtement constitué de particules sphériques comportant une surface hydrophobe,
- le milieu de séparation disposé de façon annulaire, pratiquement à la verticale, tourne autour d'un axe défini selon la direction d'écoulement du mélange à travers le milieu de séparation disposé de façon annulaire,
- on fait passer un éluant à travers le milieu de séparation disposé de manière annulaire et
- on recueille les fractions sortantes à l'extrémité du milieu de séparation disposé de manière annulaire.

2. Procédé selon la revendication 1, **caractérisé en ce que** l'agent de revêtement comporte des particules sphériques.

3. Procédé selon la revendication 1 et/ou 2, **caractérisé en ce que** le mélange contient au moins deux protéines du plasma à séparer.

4. Procédé selon une des revendications de 1 à 3, **caractérisé en ce que** le milieu de séparation disposé de façon annulaire est un milieu utilisé pour la chromatographie par échange d'ions, par perméation de gel ou par exclusion moléculaire, la chromatographie d'affinité ou la chromatographie basée sur des interactions hydrophobes.

5. Procédé selon au moins une des revendications de 1 à 4, **caractérisé en ce que** les protéines du plasma sont l'inhibiteur d'inter-α-trypsine, l'α₁-antitrypsine, l'antithrombine III, les immunoglobulines, telles que l'igG, la sérum-albumine humaine ou les glycoprotéines de préférence issues de la cascade de coagulation, ou des facteurs de coagulation sanguine dépendants de la vitamine K.

6. Procédé selon au moins une des revendications de 1 à 4, **caractérisé en ce que** les protéines du plasma sont choisies parmi les facteurs de coagulation sanguine, VIII, IX et la thrombine.

7. Procédé selon une des revendications de 1 à 6, **caractérisé en ce qu'**on effectue l'alimentation du mélange, la séparation des protéines du plasma et le fractionnement de manière continue.

8. Procédé selon une des revendications de 1 à 7, **caractérisé en ce qu'**on régénère et on met en équilibre le milieu de séparation de manière continue au cours de la séparation des protéines de plasma.

9. Procédé selon une des revendications de 1 à 8, **caractérisé en ce qu'**en utilisant en tant que milieu de séparation un matériau d'adsorption chromatographique, on fait passer au moins deux éluants différents en même temps au travers du milieu de séparation disposé de manière annulaire.

10. Procédé selon une des revendications de 1 à 9, **caractérisé en ce qu'**on utilise au moins deux milieux de séparation différents disposés en couches.

11. Procédé selon une des revendications de 1 à 10, **caractérisé en ce qu'**en tant que milieu de séparation, on utilise un matériau polymère formant un bloc compact.
